Europäisches Patentamt

**European Patent Office**

Office européen des brevets

(19)

(11) Publication number: **0 052 512**
**B1**

(12) # EUROPEAN PATENT SPECIFICATION

(45) Date of publication of patent specification: **10.06.87**

(51) Int. Cl.⁴: **A 61 B 5/04**

(21) Application number: **81305431.9**

(22) Date of filing. **17.11.81**

(54) Electrocardiographic means for detecting potential ventricular tachycardia.

(30) Priority: **18.11.80 US 208219**

(43) Date of publication of application:
**26.05.82 Bulletin 82/21**

(45) Publication of the grant of the patent:
**10.06.87 Bulletin 87/24**

(84) Designated Contracting States:
**AT BE CH DE FR GB IT LI NL SE**

(56) References cited:
**US-A-4 279 258**

**MEDICAL & BIOLOGICAL ENGINEERING &
COMPUTING, vol. 18, no. 3, May 1980,
STEVENAGE HERTS (GB), I.C. CHIEN et al.:
"Computer methods for analysing the high-
frequency electrocardiogram", pages 303-312**

**MEDICAL & BIOLOGICAL ENGINEERING &
COMPUTING, vol. 17, no. 4, July 1979,
STEVENAGE HERTS (GB), G.J.H. UIJEN et al.:
"Accuracy of QRS detection in relation to the
analysis of high-frequency components in the
electrocardiogram", pages 492-502**

**Excerpta Medica, ed. by E. Sandoe et al.,
Amsterdam 1978, Drs. Uther et al. "The
detection of ...", pp. 80-82**

(73) Proprietor: **UNIVERSITY PATENTS, INC.
537 Newtown Avenue
Norwalk Connecticut 06851 (US)**

(72) Inventor: **Simson, Michael Byron
107 Wexford Drive
Cherry Hill New Jersey (US)**

(74) Representative: **Barnard, Eric Edward et al
BROOKES & MARTIN High Holborn House 52/54
High Holborn
London WC1V 6SE (GB)**

EP 0 052 512 B1

Courier Press, Leamington Spa, England.

# 0 052 512

**Description**

Field of the invention

This invention relates to electrocardiography and more particularly, to a method of, and apparatus for analysing electrocardiographic signals.

Background to the invention

Sudden death from acute arrhythmia is a major risk in the first few hours after a myocardial infarction. During the first few days, the incidence of ventricular arrhythmia is approximately 90%. The percentage of arrhythmias decreases considerably after the first several days but still presents a substantial risk to the myocardial infarct patient. Statistically, without treatment, approximately 50% of all infarct patients will eventually die of ventricular arrhythmia.

A reproducible and consistent ability to predict a patient's propensity for lapsing into an arrhythmia is needed. Several investigators, employing signal averaging techniques, have detected, on the body surface, small high frequency potentials in the late QRS and ST-segments of electrocardiograms in patients and animals prone to ventricular tachycardia. (Uther, *et al.*: "The Detection of Delayed Activation Signals of Low Amplitude in the Vector Cardiogram of Patients with Recurrent Ventricular Tachycardia by Signal Averaging", *In Management of Ventricular Tachycardia—Role of Mexiletine*, edited by E. Sandoe *et al.*, Excerpta Medica, Amsterdam, 1978, pp. 80—82) Drs. Uther *et al.* found that these potentials did not occur in healthy young people and suggested that the presented areas of delayed myocardial depolarisation.

Obviously, if it can be shown that the high frequency signal in the late QRS of a myocardial infarct patient is common to most, if not all, infarct patients who are subject to ventricular tachycardia, an important new diagnostic tool would be available. Technically, however, it is extremely difficult to isolate accurately high frequency signals late in the QRS complex. A filter must be used to eliminate the lower frequency portions. Unfortunately, substantially all filters "ring" for a period of time after application of the relatively high energy, initial portion of the QRS waveform. This ringing effectively hides any low amplitude, high frequency portions late in the QRS.

Accordingly, it is an object of this invention to provide improved electrocardiographic signal analysis.

In a large clinical trial supervised by the inventor, using electrocardiographic analysis which will be hereinbelow described, it has been found that 92% of postmyocardial infarct patients who are subject to ventricular tachycardia, do, indeed exhibit a distinctive high frequency signal tail in their late QRS signal. This signal is present in only 7% of post infarct patients who are free of ventricular tachycardias. In addition, it was found that a patient subject to ventricular tachycardia will exhibit a QRS signal of substantially longer duration than patients without ventricular tachycardia.

Summary of the invention

As is known from the Uther et al article the present invention relates to a method of analysing electrocardiographic signals taken from a body to determine the quantity of high frequency energy in the late portion of a QRS signal, including the steps of separating at least one QRS signal from said electrocardiographic signals and sampling the QRS signal at periodic time segments where each segment has a value representative of the analog value of the QRS signal at the sampling time.

The invention is characterised by further steps of applying a portion of time segments, comprising at least the late portion of the QRS signal, in a reverse time order to a high pass filter; and·determining the quantity of high frequency energy in the late portion of the QRS signal from the output of said filter.

The present invention also provides an apparatus for analysing electrocardiographic signals to determine the level of high frequency energy in the late portion of a QRS signal, the apparatus including means for converting X, Y and Z lead electrocardiograph input signals taken from different parts of a body to digital valued time segments; means for examining said X, Y and Z digital valued time segments and selecting therefrom the QRS waveform portions thereof; and means for signal averaging a multiplicity of the selected QRS waveforms for each of the X, Y and Z inputs and providing composite digital X, Y and Z QRS waveforms, as is known from the Uther et al article. The apparatus is characterised by high pass filter means; means for applying to said high pass filter means, in reverse time order, the late portion of each said digital X, Y and Z waveforms; and means for comparing the output of said high pass filter means with a value representing a predetermined level of high frequency energy to obtain an indication of the presence of high frequency, low level energy components in said filter means output during said late portions.

As appears hereinafter in a preferred apparatus each of a patient's X, Y and Z electrocardiographic signals are converted from analog to digital values, processed to select only normal or typical QRS waveforms, and signal averaged over several hundred beats to obtain a relatively noise-free composite QRS. The latter portions of the X, Y and Z digital QRS signals are then applied in reverse time order to a digital high pass filter. The reverse time processing enables the ringing artifact to be eliminated from the filter's output. The resulting filtered outputs are combined to create a composite filtered QRS, examined, and the last 40 milliseconds of the filtered composite is isolated and measured to obtain an indication of the level of high energy content. The initial portion of the QRS waveform is also processed in a forward direction to obtain an indication of its total duration.

2

Further features and advantages of the invention will become more readily apparent from the following detailed description, when taken in conjunction with the accompanying drawings.

Brief description of the drawings

Fig. 1 is a simplified functional, block diagram of an embodiment of the invention.

Fig. 2 is a trace-showing of the signal-averaged QRS portion of a patient's electrocardiogram.

Fig. 3 is a simplified flowchart of a program utilised to implement a template selection and signal averaging routines.

Fig. 4 is a simplified flowchart of a program for implementing a high pass digital filter.

Fig. 5 is a simplified flowchart of a program for determining the last 40 millisecond portion of a filtered QRS and determining its level of high energy content.

Description of the preferred embodiment

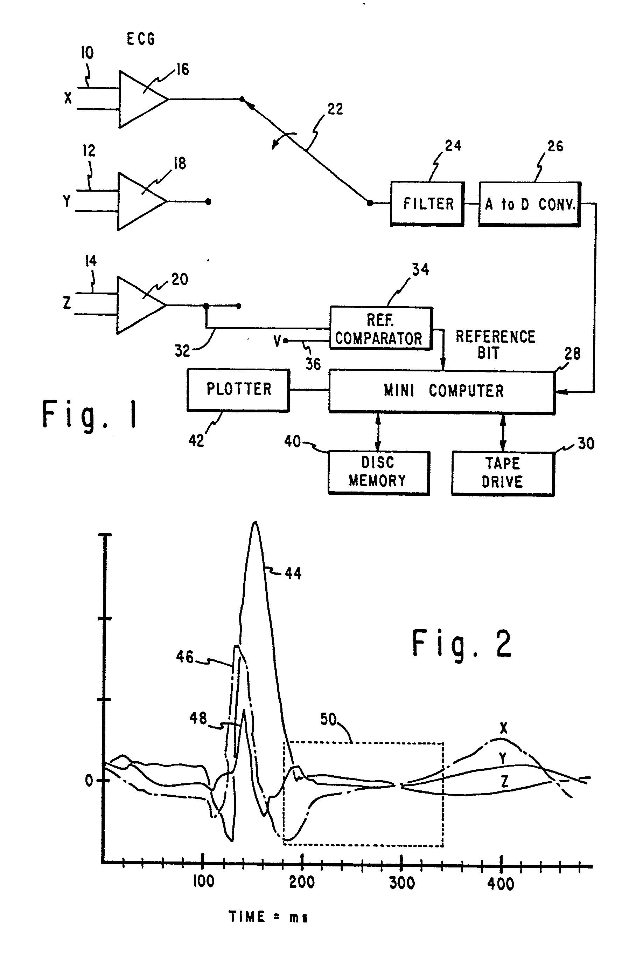

Referring now to Fig. 1, there is shown a simplified, functional, block diagram of an apparatus constructed in accordance with the invention. Each of leads 10, 12, and 14 is a bipolar electrocardiographic lead. The X lead is applied to the patient's mid-axillary line at the fourth intercostal space (under the left arm between the fourth and fifth ribs). The Y electrodes are placed at the superior aspect of the sternum and the proximal left leg. The Z electrode is at the "$V_2$" position (left of sternum at the nipple line), and the other is directly posterior. Each of the respective X, Y, and Z leads (10, 12, and 14) is fed respectively to ECG amplifiers 16, 18, and 20 (Analog Devices Model 283J isolation amplifier). The output of each amplifier is passed to a switch contact, through switch 22, and to low pass filter 24. Filter 24 characteristically attenuates all signals above 250 Hz. The output from filter 24 is fed to an analog to digital converter 26 which samples the incoming voltage every millisecond and converts it to a 12-bit binary signal. (An Analog Devices Ad572 was employed and used at a sample rate of 1,000 samples per second). The time segment outputs from A to D converter 26 are fed to minicomputer 28, which then stores the data on tape drive 30 (a Hewlett Packard 9825 desktop minicomputer was used).

The X, Y, and Z ECG signals are sequentially connected to filter 24 and A to D converter 26 by the operation of switch 22. The output from each is sampled for 133 seconds to obtain the necessary continuum of recorded signals. The output from Z ECG amplifier 20 is fed, in addition, via conductor 32 to a reference comparator 34. Also applied to reference comparator 34 is a voltage, via conductor 36 which sets the comparison level. When the QRS portion of the ECG signal appears on line 32, and it passes through voltage V, the reference comparator generates a reference bit which is recorded along with the corresponding time segment output of A to D converter 26. This reference bit enables all QRS waves to be overlaid, one on another, for selection and averaging purposes (to be discussed hereinbelow). Also connected to minicomputer 38 are disc memory 40 and plotter 42, whose functions also will be hereinafter discussed.

Referring now to Fig. 2, ECG waveforms from the X, Y, and Z leads (as seen at the outputs of amplifier 16, 18, and 20) are respectively shown. Waveforms 44, 46, and 48 are the respective QRS portions of a patient's ECG as sensed by each of ECG leads 14, 10, and 12, respectively. It is the portion of the QRS waves enclosed by box 50 wherein it has been found that high frequency anomalies occur, which are indicative of an infarct patient's propensity toward ventricular tachycardia. Before the portion of the signal, appearing in box 50, can be examined, however, a number of preprocessing steps must be accomplished.

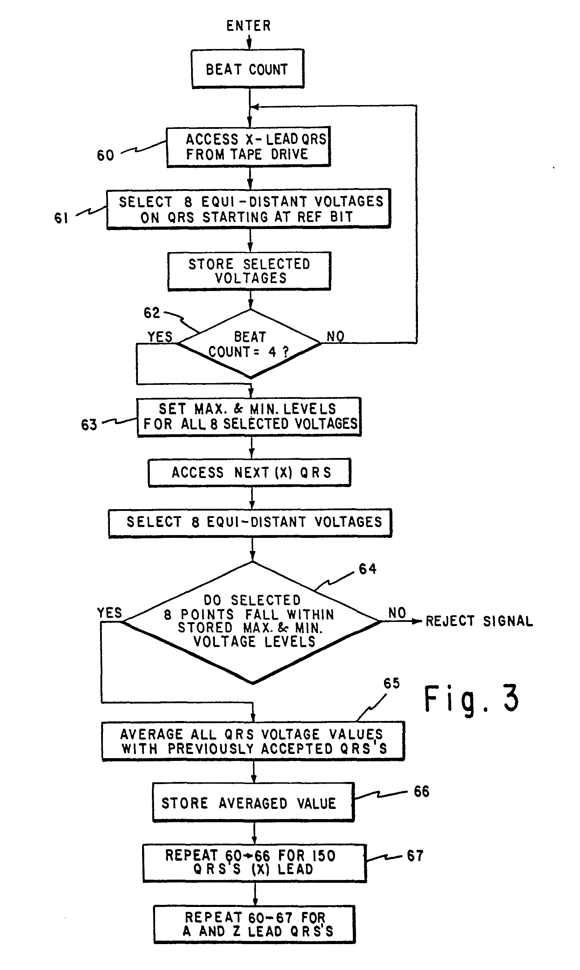

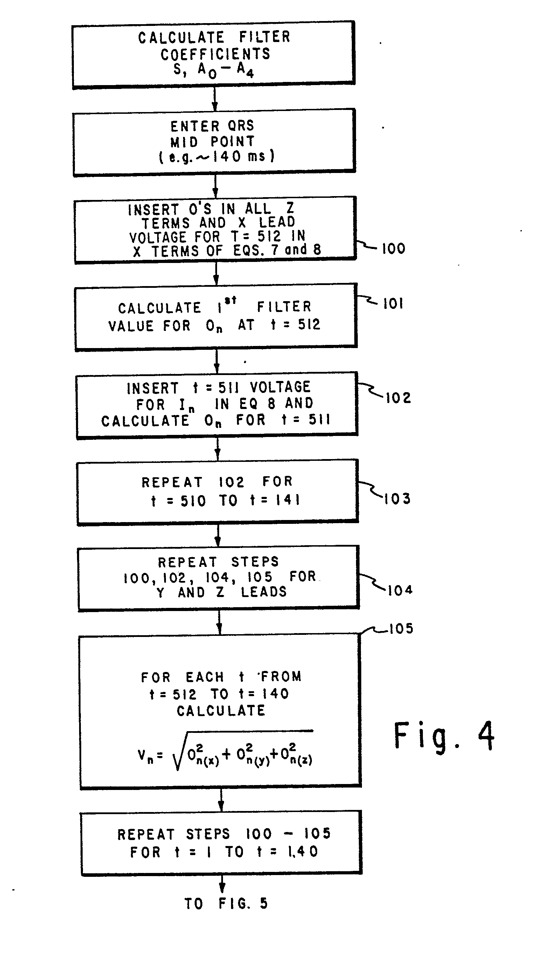

Referring now to Fig. 3, there is illustrated a simplified flowchart of a computer routine utilized to implement a "template" selection and signal averaging routines. Initially, a single beat, including a QRS, is accessed from the tape drive and placed in a buffer register, as illustrated by block 60. The reference bit is here employed to grossly acquire the location of the QRS. Subsequently, eight equidistant voltage points on the QRS, starting with one at the reference bit and ending with one at 128 milliseconds, are selected and stored (box 61). This process continues for four QRS counts, as indicated by decision diamond 62, and enables the establishment of the initial template against which succeeding QRS signals will be tested. After the fourth QRS signal is stored, the maximum and minimum voltage values for each of the eight voltage points on the four recorded QRS waveforms are tabulated and become the initial template (box 63). Then, the next QRS signal is selected, its eight voltage points are determined and stored, and, as indicated in decision diamond 64, each point is selectively tested against the stored maxima and minima to determine whether it falls within or without the respective values. If it is found that there is a mismatch in any one of the eight points, the signal is rejected as not being a QRS or being some other artifact which is not of interest. If all eight points fall within the maxima and minima, the waveform is accepted as a QRS, and its 512 voltage points, spanning the accepted QRS, are then averaged with the corresponding 512 points of the previously stored QRS signals (box 65), and the resulting averaged value stored in disc memory 50 (box 66). This subroutine is repeated for 150 QRS's which are subsequently passed through the template, averaged, and then stored to accomplish a composite-averaged QRS wave for the X lead. The template voltage minimum and maximum test points may be updated during the processing to assure accurate QRS selection. The same subroutine is then repeated for the Y and Z leads, and the averaged values for each of the composite Y and Z QRS signals also are respectively stored in disc memory 40.

The above processing greatly reduces the noise inherent in the QRS signal—by the square root of the number of averaged beats—and provides three averaged QRS waveforms which are relatively noise-free and suitable for subsequent processing. Approximately 150 beats per lead are signal-averaged and recorded. At this point, the recorded QRS waveforms may be plotted out on plotter 42 for examination by the physician. The plot also enables the physician to pick out the midpoint of the QRS for the subsequent filtering step.

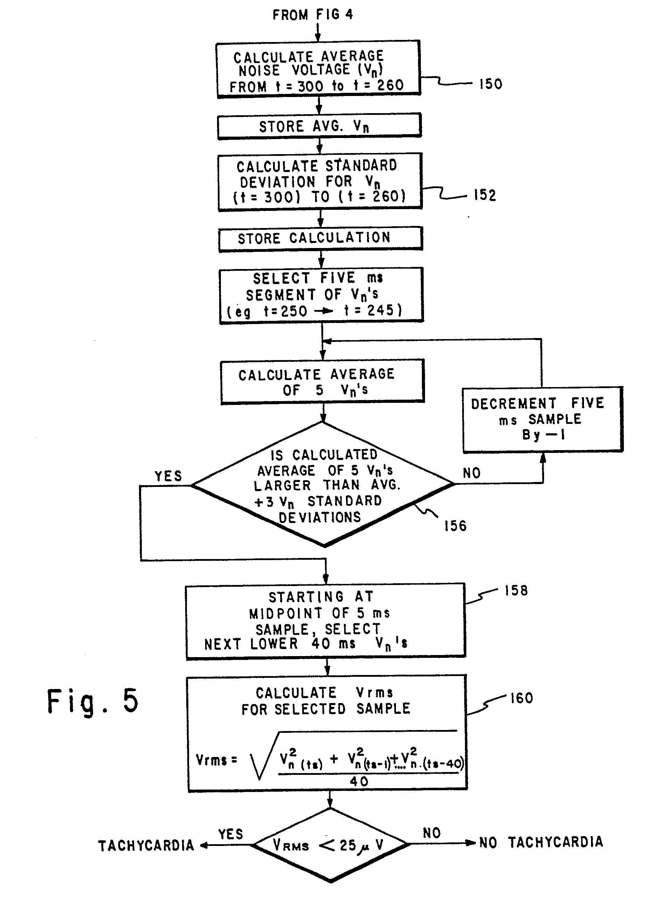

Referring now to Fig. 4, a flowchart is shown which describes, in simplified detail, the digital filtering employed to further analyze the averaged QRS waveforms. Digital filters are well-known in the art and will not be described here in any substantial detail. Reference is made, however, to two recognized works (*i.e.,* *Digital Signal Analysis* by S. D. Stearns, Hayden Book Company, Inc., (1975) pp. 182—222; and *Digital Signal Processing* by Oppenheim and Schafer, Prentice-Hall, Inc., (1975) pp. 195—282), the contents of both of which are incorporated herein by reference. The aforementioned excerpts teach, in detail, the methods for designing various digital implementations of analog filters. In this instance, the digital filter design employed was a four-pole, high pass, Butterworth design. While the Butterworth filter is only one of a number which can be employed, it does exhibit a maximally flat response above the corner frequency (in this case 25 Hz). It continuously attenuates signals below the corner frequency and provides reasonably smooth transitions between frequencies passed to those not passed.

Referring now to Fig. 4, the first operation which must be performed is to calculate the filter coefficients S, and $A_0$ to $A_4$. Each of the following coefficients has the following equation:

$$S = T_1^4/Q \qquad \text{(equation 1)}$$

$$A_0 = 1 \qquad \text{(equation 2)}$$

$$A_1 = (4T_1^4 + 2AT_1^3 - 2AT_1 - 4)/Q \qquad \text{(equation 3)}$$

$$A_2 = (6T_1^4 - 2BT_1^2 + 6)/Q \qquad \text{(equation 4)}$$

$$A_3 = (4T_1^4 - 2AT_1^3 + 2AT_1 - 4)/Q \qquad \text{(equation 5)}$$

$$A_4 = (T_1^4 - AT_1^3 + BT_1^2 - AT_1 + 1)/Q \qquad \text{(equation 6)}$$

where:

$T_0 = 500 - Fc$, where Fc is the corner frequency of the filter (i.e., 25 Hz).

$$T_1 = \mathrm{Tan}\left(\frac{\pi T_0}{1,000}\right)$$

$$A = 2\cos\left(\frac{\pi}{8}\right) + 2\sin\left(\frac{\pi}{8}\right)$$

$$B = 2 + 4\cos\left(\frac{\pi}{8}\right) \cdot \sin\left(\frac{\pi}{8}\right)$$

$$Q = T_1^4 + AT_1^3 + BT_1^2 + AT_1 + 1$$

Each of equations 1—6 is calculated by inserting the corner frequency (Fc) of 25 Hz and calculating as above shown.

Next, the midpoint of X lead QRS is entered (*i.e.,* it may be selected by examination of the plotted QRS or automatically by determining the most positive time voltage segment, *e.g.,* 140 milliseconds). Subsequently the following equations are solved to carry out the filter function:

$$O_n' = S(I_n - 4X_1 + 6X_2 - 4X_3 + X_4) \qquad \text{(equation 7)}$$

$$O_n = O_n' - A_1Z_1 - A_2Z_2 - A_3Z_3 - A_4Z_4) \qquad \text{(equation 8)}$$

where:

$I_n$ = input value
$O_n$ = output value

$$Z_4 = Z_3 \qquad \text{(equation 9)}$$

$$Z_3 = Z_2 \qquad \text{(equation 10)}$$

$$Z_2=Z_1 \qquad \text{(equation 11)}$$

$$Z_1=O_n \qquad \text{(equation 12)}$$

$$X_4=X_3 \qquad \text{(equation 13)}$$

$$X_3=X_2 \qquad \text{(equation 14)}$$

$$X_2=X_1 \qquad \text{(equation 15)}$$

$$X_1=I_n \qquad \text{(equation 16)}$$

To commence the filter's operation, or to reset it, zeros are inserted in all Z terms of equation 8, and the voltage time segment to be filtered ($I_n$) is inserted in all X terms of equation 7 (box 100). In this case, the last time segment voltage (t=512 ms) is employed, and equations 7 and 8 are solved for the value of $O_n$ which corresponds to an input signal $I_n$ equivalent to the voltage value at t=512 (box 101). Each of the variables in equations 7 and 8 are then reset in accordance with the equalities shown in equations 9—16 and the filter routine is repeated, starting with equation 7 and proceeding backward in time, for the next preceding voltage time segment t=511 ms. This entire process is repetitively done for all segments to t=141 ms (boxes 102 & 103). Each of the calculated filter output values is stored, and the entire process repeated for the outputs from the Y and Z leads (box 104). Subsequently, a composite voltage $V_n$ is calculated in accordance with the equation

$$V_n=\sqrt{O_{n(x)}{}^2+O_{n(y)}{}^2+O_{n(z)}{}^2} \qquad \text{(equation 17)}$$

(box 105). The entire filter process is then repeated for the voltage samples corresponding to time segments $t_1$ to $t_{140}$.

In summary, what has been achieved to this point is the backward filtering of the composite QRS waveforms from t=512 ms through t=141 ms, and the forward filtering of the composite QRS waveforms from t=1 ms to t=140 ms. The rearward filtering avoids the ringing perturbation which would have occurred had the signal been inserted into the filter in the forward manner, and enables the late portion of the QRS to be examined for a low amplitude, high frequency signal, indicative of potential ventricular tachycardia. In addition, an averaged, filtered composite of the patient's QRS is now stored and ready for further processing.

Turning now to Fig. 5, the portion of the filtered QRS which corresponds to the late section containing the potential high frequency energy of interest is to be located. This is achieved by first (box 150) selecting a 40 millisecond sample substantially after the termination of the major portion of the QRS (e.g., t=300 ms to t=260 ms) and averaging the $V_n$ values to achieve an average noise voltage for that sample. That average noise is stored, and a standard noise level deviation is calculated (box 152) employing the following equation:

$$\text{Standard deviation}=\sqrt{\frac{\sum\limits_{n=260}^{n=300} V_n{}^2 - \frac{\left(\sum\limits_{n=260}^{n=300} V_n\right)^2}{40}}{39}} \qquad \text{(equation 18)}$$

This standard noise deviation is stored, and a 5 millisecond sample of the QRS is selected (e.g., from t=250 ms to t=255 ms). The average value of the time segment voltages from t=250 ms to t=255 ms is calculated and compared to the average noise level plus three standard deviations previously determined. If the calculated value for the 5 millisecond sample does not exceed the total, the time segment is decremented by one time slot (i.e., one millisecond), and the process repeated until the selected average voltage of the sample does exceed the level of the calculated noise plus three standard deviations (decision diamond 156). This occurrence indicates that the selection process has arrived at the termination of the QRS signal (i.e., the middle time segment of the 5 millisecond sample is defined as the end of the QRS).

In order to determine whether the QRS signal has or does not have the high frequency tail referred to above, the voltage sample in the middle time segment ($t_s$) of the 5 millisecond sample is then selected as well as the next lower 39 voltage time segments (e.g., from t=225 to t=186), as shown by box 158. The root mean square value of all of these voltages is then calculated in accordance with equation 19 (box 160):

$$V_{RMS}=\sqrt{\frac{V_{n(t_s)}{}^2+V_{n(t_s-1)}{}^2+\ldots V_{n(t_s-40)}{}^2}{40}} \qquad \text{(equation 19)}$$

The RMS voltage of the 40 ms sample is then compared to 25 microvolts, and if it exceeds 25 microvolts, it is indicative that the patient is not susceptible to ventricular tachycardia; whereas, if it is less than 25 microvolts, it is indicative that the patient is subject to ventricular tachycardia. It should be understood that the high frequency component found in patients with ventricular tachycardia extends the tail of the QRS by several tens of milliseconds, but at a relatively low level. Thus, a low level measurement indicates that there is a low level, high frqeuency tail of energy appended to the QRS. If the voltage exceeds the 25 microvolt level, it is indicative that, in lieu of there being the aforementioned tail of high frequency energy, the measurement is actually being made on the major portion of the QRS signal which has high levels of high frequency energy. The results of these tests can be displayed or printed out by minicomputer 28 shown in Fig. 1 for the physician's use.

It has also been found that the width of the QRS waveform has a relationship to a patient with ventricular tachycardia. In order to measure the width of the QRS in the above system, it is only necessary to obtain an indication of the beginning of the QRS waveform, as the end of the QRS has already been determined, *i.e.*, at box 158 of Fig. 5. The initiation of the QRS is calculated in much the same manner. In specific, from t=1 to t=40, a 40-millisecond sample of noise measurements is averaged, and the standard deviation calculated. Five millisecond values are then selected and tested to determine whether the average value of each 5-millisecond sample exceeds the average noise plus three standard deviations. For the 5-millisecond sample which does exceed that level, the beginning of the QRS is then defined as the middle time segment of that 5-millisecond segment. The duration of the QRS then stretches from the middle of that segment to the end of the QRS as defined above.

The above-mentioned apparatus was employed in a substantial clinical test at the Cardiovascular Section, Hospital of the University of Pennsylvania, Philadelphia, Pennsylvania. Twenty-seven control patients and 39 patients with ventricular tachycardia were studied. All patients had had myocardial infarctions, were off anti-arrhythmic drugs, and did not have bundle branch block. The 39 patients with ventricular tachycardia had either sustained or inducible ventricular tachycardia. The QRS duration was found to be longer in patients with ventricular tachycardia, *i.e.*, 139 milliseconds±26 ms vs. 95 milliseconds±10 ms. Seventy-three percent of the patients with ventricular tachycardia had a QRS duration longer than 120 milliseconds, but none of the control group did. The filtered QRS voltage revealed that patients with ventricular tachycardia had a low amplitude and slowly declining high frequency signal at the end of the QRS. In contrast, the control group had a different high frequency energy distribution; the high frequency voltage at the end of the QRS was of larger amplitude but ended abruptly. Ninety-two percent of the patients with ventricular tachycardia had less than 25 microvolts of high frequency energy in the last 40 milliseconds of the QRS; only 7% of patients without ventricular tachycardia had less than 25 microvolts in this segment. On average, the control patients exhibited a 74 microvolt RMS level, whereas the ventricular tachycardia patients exhibited a 15 microvolt level. In summary, this study of the high frequency voltage in the late QRS identified patients with ventricular tachycardia after myocardial infarction, with a 92% sensitivity and a 93% specificity.

While the invention has been illustrated with respect to specific hardware, it should be understood that alternative general or specific purpose computing equipment or hard wired logic circuitry could be used in practicing the invention.

**Claims**

1. A method of analysing electrocardiographic signals taken from a body to determine the quantity of high frequency energy in the late portion of a QRS signal, includingr the steps of separating at least one QRS signal from said electrocardiographic signals and sampling the QRS signal at periodic time segments where each segment has a value representative of the analog value of the QRS signal at the sampling time, and characterised by the steps of applying a portion of the time segments, comprising at least the late portion of the QRS signal, in a reverse time order to a high pass filter; and determining the quantity of high frequency energy in the late portion of the QRS signal from the output of said filter.

2. The method of claim 1 further including the step of comparing the quantity of the high frequency energy with a predetermined level of high frequency energy.

3. The method of claim 2 further including the steps of generating a first signal if the quantity is greater than said predetermined level, and generating a second signal if the quantity is less than said predetermined level.

4. The method of claim 1, 2 or 3 wherein the separating step includes the step of separating a series of QRS signals from said electrocardiographic signals.

5. The method of claim 4 wherein the sampling step includes the step of converting the series of QRS signals to the time segments, each segment having a digital value equivalent to the analog value of said QRS signals at the time of conversion.

6. The method of claim 5 further including the step of averaging the digital values of said series of QRS signals to obtain an averaged composite QRS signal before the application thereof to said high pass filter.

7. The method of claim 6 wherein said portion of said time segments includes the last 40 milliseconds of the averaged composite QRS signal.

8. The method of claim 6 or 7 wherein said quantity of high frequency energy determined is the root means square of the values of the time segments of the late portion of the composite QRS signal.

9. The method of claim 6, 7 or 8 and further including the step of measuring the duration of said averaged composite QRS signal.

10. Apparatus for analysing electrocardiographic signals to determine the level of high frequency energy in the late portion of a QRS signal; the apparatus including means for converting X, Y and Z lead electrocardiograph input signals taken from different parts of a body to digital valued time segments; means for examining said X, Y and Z digital valued time segments and selecting therefrom the QRS waveform portions thereof; and means for signal averaging a multiplicity of the selected QRS waveforms for each of the X, Y and Z inputs and providing composite digital X, Y and Z QRS waveforms, the apparatus being characterised by high pass filter means; means for applying to said high pass filter means, in reverse time order, the late portion of each said digital X, Y and Z waveforms; and means for comparing the output of said high pass filter means with a value representing a predetermined level of high frequency energy to obtain an indication of the presence of high frequency, low level energy components in said filter means output during said late portions.

11. The apparatus as defined in claim 10 wherein said comparing means examines only a portion of the output of said filter comprising at least the last part of the late portion of the QRS signal.

12. The apparatus as defined in claim 11 wherein said portion of the output of said filter represents the filtered last 40 milliseconds of the QRS waveform.

13. The apparatus as defined in claim 12 wherein said comparing means combines selected portions of the output of said filter for each of the X, Y and Z signals and calculates the root means square value of said combined portions.

14. The apparatus as defined in claim 13 further comprising means for applying to said filter means, in forward time order, the early portion of each said digital X, Y and Z QRS waveforms; means for extracting from said filter's output the time of commencement of said QRS waveform; and means responsive to said extraction means and said comparing means for determining the width of said QRS waveform.

**Patentansprüche**

1. Verfahren zur Analyse von an einem Körper gemessenen EKG-Signalen zur Bestimmung der Größe der Hochfrequenzenergie im späten Abschnitt eines QRS-Signals mit den Schritten der Trennung von wenigstens einem QRS-Signal von den EKG-Signalen und der Abtastung des QRS-Signals in periodischen Zeitsegmenten, wobei jedes Segment einen Wert aufweist, der dem Analogwert des QRS-Signals zur Abtastzeit entspricht, gekennzeichnet durch die Schritte der Einspeisung eines Teiles der Zeitsegmente, welche zumindest den späten Abschnitt des QRS-Signals enthalten, in verkehrter Zeitfolge in ein Hochpaßfilter und der Bestimmung der Größe der Hochfrequenzenergie im späten Abschnitt des QRS-Signals aus dem Ausgangssignal dieses Filters.

2. Verfahren nach Anspruch 1, welches weiters einen Schritt des Vergleichens der Größe der Hochfrequenzenergie mit einer vorgegebenen Schwelle der Hochfrequenzenergie umfaßt.

3. Verfahren nach Anspruch 2, welches weiters die Schritte der Erzeugung eines ersten Signals, wenn die Größe größer ist als die vorbestimmte Schwelle, und die Erzeugung eines zweiten Signals umfaßt, wenn die Größe geringer ist als die vorbestimmte Schwelle.

4. Verfahren nach Anspruch 1, 2 oder 3, wobei der Abtrennschritt den Schritt einer Abtrennung einer Serie von QRS-Signalen aus den EKG-Signalen umfaßt.

5. Verfahren nach Anspruch 4, wobei der Abtastschritt den Schritt der Konvertierung der Serie von QRS-Signalen in die Zeitsegmente umfaßt, wobei jedes Segment einen digitalen Wert aufweist, welcher äquivalent dem analogen Wert der QRS-Signale zum Zeitpunkt der Konversion ist.

6. Verfahren nach Anspruch 5, welches weiters einen Schritt einer Mittelung der digitalen Werte der Serie von QRS-Signalen umfaßt, um ein mittleres gesamtes QRS-Signal vor der Einspeisung desselben in das Hochpaßfilter zu erhalten.

7. Verfahren nach Anspruch 6, wobei der Anteil der Zeitsegmente die letzten 40 Millisekunden des gemittelten gesamten QRS-Signales umfaßt.

8. Verfahren nach Anspruch 6 oder 7, wobei die Größe der gefundenen Hochfrequenzenergie der quadratische Mittelwert der Werte der Zeitsegmente des späten Abschnittes des gesamten QRS-Signales ist.

9. Verfahren nach Anspruch 6, 7 oder 8, welches weiters einen Schritt der Messung der Dauer dieses gemittelten zusammengesetzten QRS-Signales umfaßt.

10. Vorrichtung zur Analyse von EKG-Signalen zur Bestimmung der Höhe der Hochfrequenzenergie im späten Abschnitt eines QRS-Signals mit Mitteln zur Konversion von Eingangssignalen von X, Y und Z EKG-Leitungen, welche von verschiedenen Teilen eines Körpers aufgenommen werden, in Zeitsegmente mit Digitalwerten, mit Mitteln zur Untersuchung der X, Y und Z Zeitsegmente mit Digitalwerten und zur Auswahl des QRS-Kurvenanteils aus diesen und mit Mitteln zur Signalmittelung einer Vielzahl von ausgewählten QRS-Kurvenformen für jeden X, Y und Z Eingang und Lieferung von zusammengesetzten digitalen X, Y und Z QRS-Kurvenformen, gekennzeichnet durch ein Hochpaßfilter, Mittel zur Einspeisung des späten Abschnittes jeder dieser digitalen X, Y und Z Kurvenformen in dieses Hochpaßfilter in umgekehrter Zeitordnung und Mittel zum Vergleich des Ausgangssignals dieses Hochpaßfilters mit einem Wert, der eine vorgegebene Schwelle der Hochfrequenzenergie darstellt, um eine Anzeige für das Vorhandensein von Komponenten hoher Frequenz mit niedrigem Energieniveau in dem Filterausgangssignal während der letzten Abschnitte zu erhalten.

11. Vorrichtung nach Anspruch 10, wobei die Mittel zum Vergleichen nur einen Teil des Ausgangssignals des Filters untersuchen, welcher zumindest den letzten Teil des späten Abschnittes des QRS-Signales umfaßt.

12. Vorrichtung nach Anspruch 11, wobei der Teil des Filterausgangssignals die gefilterten letzten 40 Millisekunden der QRS-Kurvenform darstellt.

13. Vorrichtung nach Anspruch 12, wobei die Mittel zum Vergleichen ausgewählte Abschnitte aus dem Ausgangssignal des Filters für jedes der X, Y und Z Signale zusammenfaßt und den quadratischen Mittelwert der zusammengefaßten Abschnitte berechnet.

14. Vorrichtung nach Anspruch 13, welche weiters Mittel zur Einspeisung des frühen Abschnittes einer jeden digitalen X, Y, Z QRS-Kurvenform in richtiger Zeitordnung in das Filter, Mittel zur Bestimmung der Zeit des Beginns der QRS-Kurvenform aus dem Ausgangssignal des Filters und Mittel umfaßt, die auf die Mittel zur Gewinnung und zum Vergleich zur Bestimmung der Breite der QRS-Kurvenform ansprechen.

## Revendications

1. Procédé d'analyse de signaux électrocardiographiques obtenus à partir d'un corps afin de déterminer la quantité d'énergie à haute fréquence dans la partie tardive d'un signal QRS, comportant les étapes de séparer au moins un signal QRS à partir desdits signaux électrocardiographiques et d'échantillonner le signal QRS en des segments de temps périodiques, avec chaque segment possédant une valeur représentative de la valeur analogique du signal QRS au temps d'échantillonnage, et caractérisé par les étapes d'appliquer une partie des segments de temps, comprenant au moins la partie tardive du signal QRS, dans un order inverse dans le temps, à un filtre passe-haut, et de déterminer la quantité d'énergie en haute fréquence dans la partie tardive du signal QRS provenant de la sortie dudit filtre.

2. Procédé suivant la revendication 1, comprenant en outre l'étape de comparer la quantité de l'énergie en haute fréquence avec un niveau prédéterminé d'énergie en haute fréquence.

3. Procédé suivant la revendication 2, comprenant en outre les étapes de produire un premier signal si la quantité est supérieure audit niveau prédéterminé, et de produire un second signal si la quantité est inférieure audit niveau prédéterminé.

4. Procédé suivant la revendication 1, 2 ou 3, dans lequel l'étape de séparation comprend l'étape de séparer une série de signaux QRS à partir desdits signaux électrocardiographiques.

5. Procédé suivant la revendication 4, dans lequel l'étape d'échantillonnage comprend l'étape de convertir la série de signaux QRS en les segments de temps, chaque segment possédant une valeur numérique équivalent à la valeur analogique desdits signaux QRS au temps de conversion.

6. Procédé suivant la revendication 5, comprenant en outre l'étape de faire la moyenne des valeurs numériques de ladite série de signaux QRS pour obtenir un signal QRS composé moyen avant son application audit filtre passe-haut.

7. Procédé suivant la revendication 6, dans lequel ladite partie desdits segments de temps comprend les 40 dernières millisecondes du signal QRS composé moyen.

8. Procédé suivant la revendication 6 ou 7, dans lequel ladite quantité d'énergie en haute fréquence déterminée est la valeur efficace des valeurs des segments de temps de la partie tardive du signal QRS composé.

9. Procédé suivant la revendication 6, 7 ou 8, et comprenant en outre l'étape de mesurer la durée dudit signal QRS composé moyen.

10. Appareil pour analyser des signaux électrocardiographiques afin de déterminer le niveau d'énergie en haute fréquence dans la partie tardive d'un signal QRS, l'appareil comprenant des moyens pour convertir des signaux d'entrée cardiographiques de fils X, Y et Z obtenus à partir de différentes parties d'un corps en des segments de temps évalués numériquement; des moyens pour examiner lesdits segments de temps évalués numériquement X, Y et Z et sélectionner à partir de ceux-ci leurs parties de formes d'ondes QRS, et des moyens pour établir la moyenne de signaux d'une multiplicité des formes d'ondes QRS sélectionnées pour chacune des entrées X, Y et Z et offrir des formes d'ondes QRS X, Y et Z numériques composées, l'appareil étant caractérisé par des moyens de filtre passe-haut; des moyens pour appliquer auxdits moyens de filtre passe-haut dans un ordre inverse dans le temps, la partie tardive de chacune desdites formes d'ondes numériques X, Y et Z, et des moyens pour comparer la sortie desdits moyens de filtre passe-haut à une valeur représentant un niveau prédéterminé d'énergie en haute fréquence afin d'obtenir une indication de la présence de composantes à haute fréquence et bas niveau dans ladite sortie des moyens de filtre pendant lesdites parties tardives.

11. Appareil suivant la revendication 10, dans lequel lesdits moyens de comparaison n'examinent qu'une partie de la sortie dudit filtre comprenant au moins la dernière portion de la partie tardive du signal QRS.

12. Appareil suivant la revendication 11, dans lequel ladite partie de la sortie dudit filtre représente les 40 dernières millisecondes filtrées de la forme d'onde QRS.

**0 052 512**

13. Appareil suivant la revendication 12, dans lequel lesdits moyens de comparaison combinent des parties sélectionnées de la sortie dudit filtre pour chacun des signaux X, Y et Z et calculent la valeur efficace desdites parties combinées.

14. Appareil suivant la revendication 13, comprenant en outre des moyens pour appliquer auxdits moyens de filtre, dans l'ordre inverse dans le temps, la partie précoce de chacune desdites formes d'ondes QRS numériques X, Y et Z; des moyens pour extraire à partir de ladite sortie du filtre le temps de commencement de ladite forme d'onde QRS, et des moyens réagissant auxdits moyens d'extraction et auxdits moyens de comparaison afin de déterminer la largeur de ladite forme d'onde QRS.

9

Fig. 1

Fig. 2

TIME = ms

ENTER

BEAT COUNT

60 — ACCESS X-LEAD QRS FROM TAPE DRIVE

61 — SELECT 8 EQUI-DISTANT VOLTAGES ON QRS STARTING AT REF BIT

STORE SELECTED VOLTAGES

62 — BEAT COUNT = 4 ?
YES / NO

63 — SET MAX. & MIN. LEVELS FOR ALL 8 SELECTED VOLTAGES

ACCESS NEXT (X) QRS

SELECT 8 EQUI-DISTANT VOLTAGES

64 — DO SELECTED 8 POINTS FALL WITHIN STORED MAX. & MIN. VOLTAGE LEVELS
YES / NO → REJECT SIGNAL

65 — AVERAGE ALL QRS VOLTAGE VALUES WITH PREVIOUSLY ACCEPTED QRS'S

66 — STORE AVERAGED VALUE

67 — REPEAT 60→66 FOR 150 QRS'S (X) LEAD

REPEAT 60→67 FOR A AND Z LEAD QRS'S

Fig. 3

2

Fig. 4

Flowchart contents:

CALCULATE FILTER
COEFFICIENTS
$S, A_0 - A_4$

↓

ENTER QRS
MID POINT
( e.g. ~140 ms )

↓

INSERT O'S IN ALL Z
TERMS AND X LEAD
VOLTAGE FOR T = 512 IN
X TERMS OF EQS. 7 and 8 — 100

↓

CALCULATE $1^{st}$ FILTER
VALUE FOR $O_n$ AT $t = 512$ — 101

↓

INSERT $t = 511$ VOLTAGE
FOR $I_n$ IN EQ 8 AND
CALCULATE $O_n$ FOR $t = 511$ — 102

↓

REPEAT 102 FOR
$t = 510$ TO $t = 141$ — 103

↓

REPEAT STEPS
100, 102, 104, 105 FOR
Y AND Z LEADS — 104

↓ — 105

FOR EACH $t$ FROM
$t = 512$ TO $t = 140$
CALCULATE

$$V_n = \sqrt{O^2_{n(x)} + O^2_{n(y)} + O^2_{n(z)}}$$

↓

REPEAT STEPS 100 — 105
FOR $t = 1$ TO $t = 140$

↓

TO FIG. 5

0 052 512

FROM FIG 4

CALCULATE AVERAGE
NOISE VOLTAGE ($V_n$)
FROM $t = 300$ to $t = 260$ ——— 150

STORE AVG. $V_n$

CALCULATE STANDARD
DEVIATION FOR $V_n$
($t = 300$) TO ($t = 260$) ——— 152

STORE CALCULATION

SELECT FIVE ms
SEGMENT OF $V_n$'s
(eg $t = 250 \rightarrow t = 245$)

CALCULATE AVERAGE
OF 5 $V_n$'s

DECREMENT FIVE
ms SAMPLE
By $-1$

IS CALCULATED
AVERAGE OF 5 $V_n$'s
LARGER THAN AVG.
$+3 V_n$ STANDARD
DEVIATIONS          156

YES                 NO

STARTING AT
MIDPOINT OF 5 ms
SAMPLE, SELECT
NEXT LOWER 40 ms $V_n$'s ——— 158

Fig. 5

CALCULATE Vrms
FOR SELECTED SAMPLE          160

$$Vrms = \sqrt{\frac{V^2_{n\,(ts)} + V^2_{n(ts-1)} + \ldots V^2_{n\,.(ts-40)}}{40}}$$

TACHYCARDIA ← YES  $V_{RMS} < 25\,\mu V$  NO → NO TACHYCARDIA

4